# FASCICULE DE BREVET EUROPEEN

(11) **EP 3 181 177 B1**
(45) Date de publication et mention de la délivrance du brevet: **22.07.2020**
(21) Numéro de dépôt: 16205167.6
(22) Date de dépôt: 19.12.2016
(51) Int. Cl.: A61M 25/01, A61M 25/09, A61B 5/042, A61M 25/00

(54) **NÉCESSAIRE DE TRAITEMENT, DISPOSITIF DE MESURE ET UTILISATION ASSOCIÉS**
BEHANDLUNGSGERÄT, ENTSPRECHENDE MESSVORRICHTUNG UND ENTSPRECHENDE VERWENDUNG
TREATMENT KIT, ASSOCIATED MEASURING DEVICE AND ASSOCIATED USE

(30) Priorité: 18.12.2015 FR 1562850
(43) Date de publication de la demande: 21.06.2017
(73) Titulaire: Perouse Medical, 60173 Ivry le Temple (FR)
(72) Inventeur: Vin, Laëtitia, 27140 Gisors (FR); Walter, Thomas, 92500 Rueil Malmaison (FR)
(74) Mandataire: Lavoix

(56) Documents cités:
- WO-A1-2011/094631
- US-A1- 2003 144 656
- US-A1- 2008 255 475

## Description

La présente invention concerne un nécessaire de traitement comprenant un guide comportant une extrémité distale et une extrémité proximale, un dispositif de détermination de la position de l'extrémité distale du guide dans le corps d'un patient, et un cathéter destiné à être inséré dans le corps du patient.

Le document US2008/255475A1 décrit un tel nécessaire de traitement.

Un tel nécessaire est utilisé, par exemple, pour préparer la pose d'un implant d'accès veineux, telle qu'une chambre implantable, un cathéter central inséré périphériquement (PICC), un cathéter veineux central (CVC).

Il est important de pouvoir positionner précisément l'implant dans le corps du patient. En particulier, dans le domaine de l'implantation vasculaire, il est souhaitable de pouvoir positionner un cathéter de sorte que son extrémité distale arrive précisément au niveau de la jonction entre la veine cave supérieure et l'oreillette droite.

Il est connu d'utiliser un guide muni d'une sonde ECG implanté dans un cathéter isolant le guide. Le principe de la technique ECG, repose sur le fait que la forme et la hauteur de l'onde P de l'ECG est fonction de la position de l'électrode de détection, et varie significativement lorsque cette électrode est située à l'intersection entre la veine cave supérieure et l'oreillette droite. Cette position correspond à l'endroit où doit se trouver l'extrémité distale du cathéter implanté. Lors de l'implantation du cathéter dans le corps du patient, le guide dans le cathéter permet de déterminer la position du cathéter dans le corps du patient. Lorsque le cathéter est à la position désirée, le praticien mesure la longueur d'insertion du cathéter, puis le ressort et le découpe à la longueur d'insertion mesurée. Le cathéter mesurant la longueur d'insertion est ensuite réimplanté dans le corps du patient.

Cependant, un tel procédé nécessite une succession d'étapes complexes avec plusieurs implantations successives du cathéter dans le corps du patient, ce qui est fastidieux et long pour le praticien. En outre la réimplantation du cathéter augmente les risques d'infection.

Un but de l'invention est de proposer un nécessaire de traitement permettant une mise en place très précise et simple d'un cathéter en position dans un conduit sanguin et limitant les risques d'infections. La précision doit, de préférence, être suffisante pour assurer que le cathéter est à moins d'un centimètre de la zone ciblée.

A cet effet, la présente invention a pour objet un nécessaire selon la revendication 1.

Le nécessaire de traitement selon l'invention peut comprendre l'une ou plusieurs des caractéristiques des revendications 2 à 7.

L'invention a également pour objet un guide selon la revendication 8.

L'invention a également pour objet un dispositif de mesure selon la revendication 9.

Un procédé de préparation de l'implantation d'un cathéter comprend les étapes suivantes :
- fourniture d'un nécessaire de traitement tel que précédemment décrit,
- détermination de la position de l'extrémité distale du guide dans le corps du patient,
- insertion du guide jusqu'à une position désirée,
- mesure de la longueur d'insertion du guide par les graduations du guide, depuis l'extérieur du patient, le guide étant dans une position désirée,
- découpe du cathéter à une longueur déterminée en fonction de la longueur d'insertion mesurée.

Le procédé de préparation de l'implantation d'un cathéter peut comprendre l'une ou plusieurs des caractéristiques suivantes prise(s) isolément ou suivant toutes les combinaisons techniquement possibles :
- le cathéter est conservé en conditions stériles jusqu'à l'étape de découpe ;
- le procédé de préparation de l'implantation d'un cathéter comprend, en outre, l'étape suivante:
- retrait du guide avant une insertion du cathéter découpé ;
- le procédé de préparation de l'implantation d'un cathéter comprend, en outre, l'étape suivante :
- fourniture d'un introducteur comprenant un dilatateur, le guide étant apte à recevoir le dilatateur de l'introducteur, l'introducteur étant propre à recevoir le cathéter découpé,
- mise en place de l'introducteur au moyen du guide dans le corps du patient ;
- lorsque le guide est dans la position désirée, l'extrémité distale du guide est située à la jonction de la veine cave supérieure et de l'oreillette droite du patient ;
- la détermination de la position de l'extrémité distale du guide dans le corps du patient comporte une mesure d'électrocardiogramme, une modification de l'onde P mesurée lors de l'électrocardiogramme indiquant le positionnement du guide dans la position désirée.

L'invention sera mieux comprise à la lecture de la description qui va suivre, donnée uniquement à titre d'exemple, et faite en se référant aux dessins annexés, sur lesquels :
- la figure 1 est une vue schématique d'un dispositif de mesure d'un nécessaire de traitement selon l'invention, introduit en partie dans le corps d'un patient;
- la figure 2 est une vue du cathéter du nécessaire de traitement avant sa découpe;
- la figure 3 est une vue du cathéter du nécessaire de traitement après sa découpe;
- la figure 4 est une vue partiellement éclatée d'un exemple de guide;
- la figure 5 est une vue partielle d'un cathéter connecté à une chambre implantable;
- la figure 6 est une vue de côté d'un autre exemple de guide.

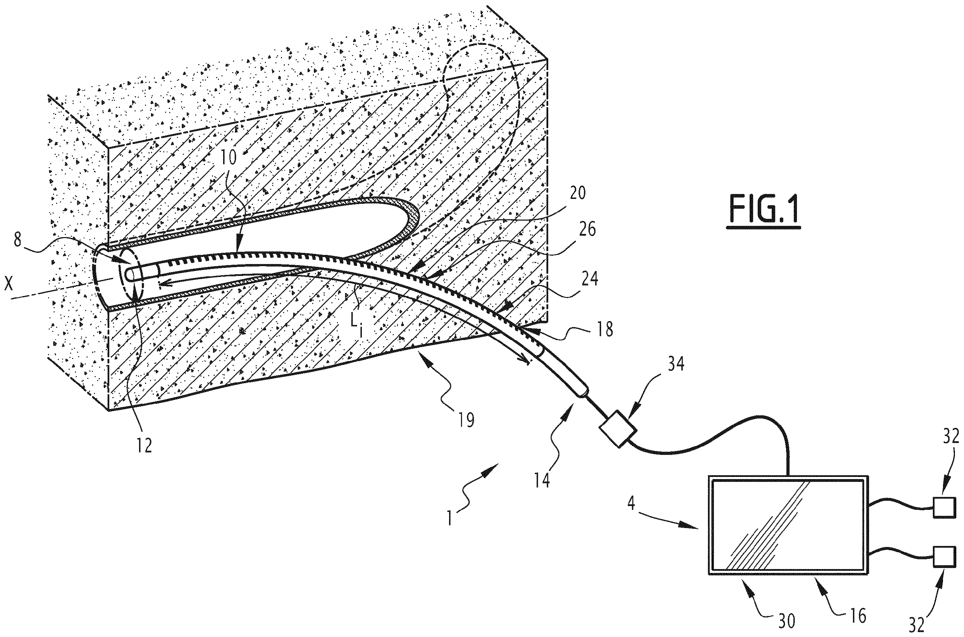

Un nécessaire de traitement 1 d'un patient est illustré sur les figures 1 à 4. Le nécessaire de traitement 1 comprend un dispositif de mesure 4 et un cathéter 6 destiné à être inséré dans le corps d'un patient.

En particulier, le cathéter 6 est destiné à être inséré dans le système sanguin, notamment au niveau d'une zone à traiter 8. La zone à traiter 8 est par exemple dans le système veineux, notamment à la jonction entre l'oreillette droite et la veine cave supérieure du patient.

Le dispositif de mesure 4 comprend un guide 10 comportant une extrémité distale 12 destinée à être introduite dans le patient et une extrémité proximale 14 destinée à rester hors du patient, et un dispositif de détermination 16 de la position de l'extrémité distale 12 du guide 10 dans le corps du patient.

Avantageusement, le nécessaire de traitement 1 comporte, en outre, une aiguille de ponction et un introducteur non représentés. Avantageusement, l'aiguille de ponction et l'introducteur utilisés sont les mêmes que dans l'état de la technique.

L'aiguille de ponction définit une lumière interne présentant un diamètre interne adapté au traitement prévu. Elle est propre à créer une ponction 18 dans la peau du patient et à permettre l'insertion du guide 10 dans le corps du patient à proximité de la zone à traiter 8 à partir de la ponction 18. La ponction 18 traverse la peau 19 du patient et débouche dans une veine, par exemple.

L'introducteur définit une lumière interne présentant un diamètre interne adapté au traitement prévu. L'introducteur est propre à recevoir le cathéter 6 découpé. En outre, l'introducteur est propre à être mis en place sur le guide 10. L'introducteur comprend un dilatateur.

Le guide 10 est allongé entre son extrémité distale 12 et son extrémité proximale 14 suivant une direction longitudinale X. La longueur du guide 10 est supérieure ou égale à la distance entre la position de la ponction 18 et la position de la zone à traiter 8. Par exemple, la longueur du guide 10 est supérieure ou égale à 60 cm.

Le diamètre externe du guide 10, mesuré transversalement à la direction longitudinale X, est strictement inférieur au diamètre interne de l'aiguille de ponction. En outre, le diamètre externe du guide 10 est strictement inférieur au diamètre interne de l'introducteur. Avantageusement, le diamètre externe du guide 10 est inférieur à 0,9 mm.

En pratique pour un nécessaire de traitement 1 destiné à l'implantation d'une chambre implantable ou un cathéter central veineux, désigné par l'acronyme anglais CVC, pour adulte, le diamètre externe du guide 10 est inférieur ou égal à 0,89 mm.

Pour un nécessaire de traitement 1 destiné à l'implantation d'une chambre implantable pour un enfant, le diamètre externe du guide 10 est inférieur ou égal à 0,46 mm. Pour un nécessaire de traitement 1 destiné à l'implantation d'un PICC pour adulte, le diamètre externe du guide 10 est inférieur ou égal à 0,46 mm. Pour un nécessaire de traitement 1 destiné à l'implantation d'un PICC pour enfant, le diamètre externe du guide 10 est inférieur ou égal à 0,36 mm.

Le guide 10 comporte une portion centrale 20 entre l'extrémité distale 12 et l'extrémité proximale 14. La portion centrale 20 est couverte d'un matériau isolant électriquement.

Le guide 10 comporte une âme 22 en matériau conducteur électriquement, par exemple en métal. L'âme 22 est allongée selon la direction longitudinale X. Par exemple l'âme 22 comporte une membrure interne 25 et un ressort 23 recouvrant la membrure 25. Le ressort 23 assure une flexibilité à l'âme 22.

Le guide 10 comporte une gaine externe 24 recouvrant l'âme 22 sur la portion centrale 20. La gaine externe 24 est en matériau isolant électriquement. Par exemple, la gaine externe 24 est en plastique et comprend du polytétrafluoroéthylène (PTFE) ou un autre matériau isolant.

Le guide 10 est gradué entre son extrémité distale 12 et son extrémité proximale 14. Les graduations 26 du guide 10 sont visibles depuis la surface externe du guide 10 et permettent de mesurer une longueur d'insertion Lᵢ du guide 10 dans le corps du patient.

Dans l'exemple, les graduations du guide 10 sont disposées sur la gaine externe 24. En variante, les graduations 26 du guide 10 sont disposées sur l'âme 22 du guide 10 et la gaine externe 24 est transparente.

Les graduations 26 du guide 10 sont régulièrement réparties le long de la direction longitudinale X du guide 10. Par exemple, les graduations 26 du guide 10 sont réparties tous les centimètres. Dans une variante, les graduations 26 du guide sont réparties tous les demi-centimètres.

La surface extérieure du guide 10 au niveau des extrémités distale 12 et proximale 14 est conductrice électriquement. L'extrémité distale 12 et l'extrémité proximale 14 du guide 10 ne sont pas recouvertes par la gaine 24 isolante. L'extrémité distale 12 et l'extrémité proximale 14 sont en matériau conducteur électriquement, par exemple dans le même matériau que l'âme. Par exemple, l'extrémité distale 12 et l'extrémité proximale 14 sont recouvertes d'un revêtement conducteur.

Lorsque le guide 10 est dans le corps du patient, l'extrémité distale 12 du guide 10 est ainsi propre à capter le potentiel électrique au voisinage de sa position. L'âme 22 permet la transmission de ce potentiel jusqu'à l'extrémité proximale 14 du guide 10. La gaine externe 24 isolante évite la pollution du potentiel capté pendant la transmission jusqu'à l'extrémité proximale du guide 10.

Le dispositif de détermination 16 est propre à déterminer la position de l'extrémité distale 12 du guide 10 dans le corps d'un patient. Le dispositif de détermination 16 de la position de l'extrémité distale 12 est dans l'exemple un dispositif d'électrocardiogramme désigné par l'acronyme ECG. Le dispositif de détermination 16 de la position de l'extrémité distale 12 du guide 10 comprend un moniteur 30, au moins une électrode de référence 32 et une sonde d'électrocardiogramme 34.

Chaque électrode de référence 32 est propre à capter un potentiel électrique à distance de la zone à traiter 8. Chaque électrode de référence est destinée à être placée à l'écart de la ponction 18 sur la peau 19 du patient. Dans l'exemple, le dispositif de détermination 16 comporte deux électrodes de références 32 destinées à être placées à l'écart de la ponction 18 sur la peau 19 du patient.

La sonde d'électrocardiogramme 34 du dispositif de détermination 16 est connectée électriquement à l'extrémité proximale 14 du guide 10. La sonde d'électrocardiogramme 34 est propre à mesurer un signal mesuré dépendant de la position de l'extrémité distale 12 du guide 10.

L'extrémité proximale 14 du guide 10, en matériau conducteur électriquement est connectée à la sonde 34 par exemple à l'aide d'une pince. En variante, la portion centrale 20 du guide 10 est enroulée dans un dévidoir et l'extrémité proximale 16 du guide 10 est connectée à la sonde par une électrode en contact avec du sérum physiologique placé dans le dévidoir du guide 10.

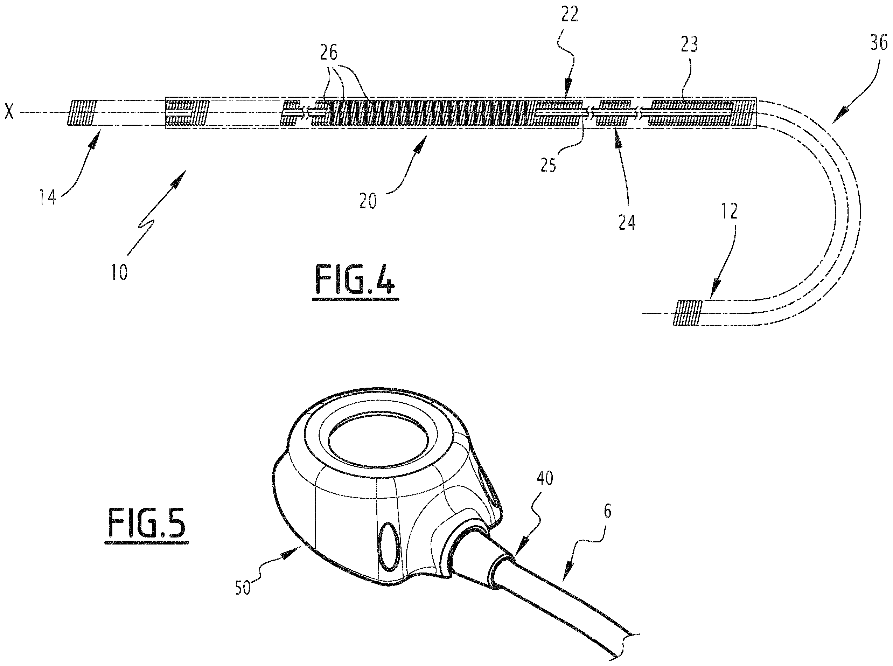

Dans l'exemple sur la figure 4, au voisinage de l'extrémité distale 12du guide 10, le guide 10 présente une courbure 36. Le rayon de courbure R est par exemple égal à 3 mm. Cette courbure 36 facilite le cheminement du guide 10 dans la veine. La portion centrale 20 isolée par la gaine 24 est sur la partie rectiligne avant la courbure 36. L'extrémité proximale 14 non isolée par la gaine mesure par exemple 2 cm. L'extrémité proximale 12 non isolée par la gaine 10 comporte la zone de la courbure 36.

Le moniteur 30 est propre à afficher une mesure d'électrocardiogramme à partir des signaux captées par la sonde 34 et/ou par les électrodes de références 32. En outre avantageusement, le moniteur 30 est propre à déterminer la forme et la hauteur de l'onde P à partir de la mesure.

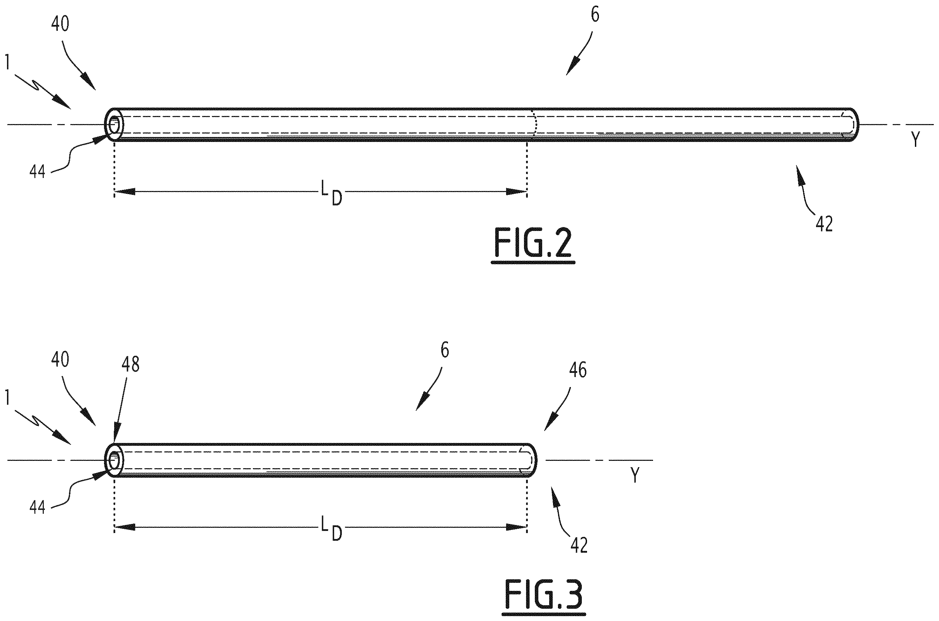

Le cathéter 6 est représenté sur la figure 2 avant sa découpe et sur la figure 3 après sa découpe.

Le cathéter 6 est destiné à être inséré dans le corps du patient. Le cathéter 6 comporte une extrémité proximale 40 et une extrémité distale 42.

Le cathéter est allongée entre son extrémité distale 42 et son extrémité proximale 40 suivant une direction d'allongement Y.

Le cathéter 6 présente généralement une longueur supérieure à 40 cm et comprise entre 50 cm et 80 cm. Il présente un diamètre compris entre 1 mm et 5 mm.

Le cathéter 6 présente une lumière interne 44 étendue suivant la direction d'allongement Y et débouchant par l'extrémité distale 42 et par l'extrémité proximale 40 du cathéter 6.

Le diamètre de la lumière interne 44, mesuré transversalement à l'axe d'allongement Y, du cathéter 6 est adaptée au traitement prévu. Par exemple le diamètre de la lumière interne 44 du cathéter 6 est compris entre 0,5 mm et 2 mm.

Dans une variante, le cathéter 6 présente une pluralité de lumières internes 44, par exemple deux ou trois lumières internes 44, étendues suivant la direction d'allongement Y et débouchant par l'extrémité distale 42 et par l'extrémité proximale 40 du cathéter 6.

Le cathéter 6 est propre à être inséré dans le corps du patient, à la place du guide 10, notamment après sa découpe.

Notamment, le cathéter 6 est propre, par exemple, à être inséré dans l'introducteur. Ainsi, le diamètre externe du cathéter 6, mesuré transversalement à l'axe d'allongement Y, est inférieur au diamètre interne de l'introducteur.

Le cathéter 6 est propre à être découpé par son extrémité distale 42. En variante ou en complément, le cathéter 6 est propre à être découpée par son extrémité proximale 40.

Le cathéter 6 est propre à être découpé à une longueur déterminée L_{D} en fonction de la longueur d'insertion Lᵢ du guide 10.

Après sa découpe, une des extrémités distale 42 ou proximale 40 du cathéter a été tronquée. Le cathéter présente une extrémité tronquée 46 et une extrémité non tronquée 48. La lumière interne 44 du cathéter débouche par l'extrémité tronquée 46 et par l'extrémité non tronquée 48. En variante, les deux extrémités du cathéter 6 sont tronquées. Dans l'exemple représenté sur la figure 3, le cathéter 6 est découpé par son extrémité distale 42. La distance entre les deux extrémités 46, 48 du cathéter après sa découpe est égale à la longueur déterminée L_{D} en fonction de la longueur d'insertion Lᵢ du guide 10.

Dans un exemple illustré sur la figure 5, le cathéter 6 comporte, en outre, une chambre implantable 50 raccordée à son extrémité proximale 40. La lumière interne 44 du cathéter 6 débouche dans la chambre implantable 50.

Dans une variante, l'extrémité proximale 40 du cathéter 6 est raccordée à l'embase d'un cathéter central inséré périphériquement désigné par l'acronyme anglais « PICC ».

Dans une variante, l'extrémité proximale 40 du cathéter 6 est raccordée à l'embase d'un cathéter veineux central désigné par l'acronyme « CVC ».Le fonctionnement du nécessaire de traitement 1 selon l'invention va maintenant être décrit en regard des figures 1 à 3.

Le nécessaire de traitement 1 est fourni. Une ponction 18, par exemple une ponction veineuse est effectuée, par exemple, par l'aiguille de ponction.

Les électrodes de références 32 sont placées à distance de la ponction 18 sur le corps du patient.

Une fois, la ponction 18 veineuse effectuée, le guide 10 est introduit dans l'aiguille de ponction par son extrémité distale 12 sans introduire le cathéter 6. Le guide 10 chemine dans la veine à partir de l'aiguille de ponction. Le guide 10 est déplacé dans l'aiguille de ponction et dans la veine par l'opérateur.

La position de l'extrémité distale 12 du guide 10, dans le corps du patient, est déterminée au moyen du dispositif de détermination. Par exemple, la détermination de la position de l'extrémité distale 12 est mise en œuvre de façon continue pendant le déplacement du guide 10.

La détermination de la position de l'extrémité distale 12 du guide 10 dans le corps du patient comporte une mesure d'électrocardiogramme. Par exemple, l'opérateur vérifie la forme de l'onde P relevée sur le moniteur 30.

Le guide 10 est inséré jusqu'à une position désirée. Par exemple, lorsque le guide 10 est dans la position désirée, l'extrémité distale du guide 10 est située à la jonction de la veine cave supérieure et de l'oreillette droite du patient. Une modification de l'onde P mesurée lors de l'électrocardiogramme indique le positionnement du guide 10 dans la position désirée. Dès que la forme de l'onde P sur le moniteur varie brutalement pendant le déplacement du guide 10, l'opérateur arrête de déplacer le guide 10.

Lorsque le guide 10 est dans la position désirée, la longueur d'insertion Lᵢ du guide 10 ainsi mesurée hors du corps du patient à l'aide des graduations 26 du guide 10 du dispositif de mesure 4.

La longueur d'insertion Lᵢ du guide 10 est établie en mesurant à l'extérieur du patient à partir de la ponction 18, les graduations 26 du guide 10 qui sont visibles hors du patient entre la ponction 18 et l'extrémité proximale 14 du guide 10.

L'utilisation de la longueur d'insertion Lᵢ préalablement mesurée pour découper, hors du corps du patient, un cathéter 6 destiné à être inséré dans le corps du patient va maintenant être décrite.

Le cathéter 6 est maintenu en conditions stériles hors du corps du patient jusqu'à sa découpe.

Une longueur de découpe L_{D} du cathéter est déterminée par l'opérateur à partir de la longueur d'insertion Lᵢ mesurée précédemment en fonction des caractéristiques du traitement prévu.

Par exemple, la longueur déterminée L_{D} de découpe est égale à la longueur d'insertion Lᵢ. Ainsi, si le cathéter 6 est placé à la place du guide 10, son extrémité proximale 40 sera au niveau de la ponction 18 et son extrémité distale 42 sera dans la position désirée sur la zone à traiter 8.

En variante, la longueur déterminée L_{D} de découpe est égale à la somme de la longueur d'insertion Lᵢ et d'une longueur prédéterminée pour que l'extrémité proximale 40 du cathéter 6 dépasse du corps du patient depuis la ponction 18, par exemple, afin de faciliter des manipulations de l'opérateur.

En variante, la longueur déterminée L_{D} de découpe est égale à la différence entre la longueur d'insertion Lᵢ et une longueur prédéterminée, par exemple, pour que le cathéter 6 soit raccordé à une chambre implantable 50 sous la ponction 18.

Le cathéter 6 est découpé à la longueur L_{D} de découpe déterminée en fonction de la longueur d'insertion Lᵢ mesurée.

Le cathéter 6 est, par exemple, découpé par son extrémité distale 42. Ainsi l'extrémité proximale 40 de cathéter 6 peut être raccordée à une chambre implantable 50 avant la découpe du cathéter 6, tel qu'illustré par la figure 5.

Il est ainsi possible de disposer de chambres implantables 50 préassemblées sur des cathéters 6 de longueur standard, et de découper l'extrémité distale 42 du cathéter 6, tout en maintenant raccordée la chambre implantable 50. Ceci simplifie considérablement le travail du praticien et garantit une connexion robuste entre la chambre implantable 50 et le cathéter 6.

En variante, le cathéter 6 est découpé par son extrémité proximale 40.

L'opérateur retire l'aiguille de ponction en laissant le guide 10 en position.

L'introducteur est mis en place au moyen du guide 10 dans le corps du patient. Lors de la mise en place de l'introducteur, l'introducteur est placé de sorte à ce que le guide 10 soit dans la lumière de l'introducteur. L'introducteur chemine en suivant le guide jusqu'à atteindre la position désirée.

En outre, le guide 10 est retiré du corps du patient depuis la ponction 18 par l'opérateur avant l'insertion du cathéter 6 découpé.

Avantageusement, les électrodes de référence 32 sont laissées sur le corps du patient et la sonde 34 est déconnectée du guide 10 puis positionnée sur le corps du patient. Cela permet à l'opérateur de continuer l'opération avec un monitorage d'électrocardiogramme standard.

L'opérateur retire le dilatateur de l'introducteur.

Après la découpe du cathéter 6, l'opérateur met en place le cathéter 6 dans l'introducteur en insérant l'extrémité distale 42 du cathéter 6 dans l'introducteur. Le cathéter 6 chemine dans l'introducteur, jusqu'au voisinage de la zone à traiter 8. Comme le cathéter 6 mesure précisément la longueur déterminée L_{D}, l'introduction du cathéter 6 découpée permet sa mise en place de sorte à ce que son extrémité distale 42 soit positionnée à la position désirée.

L'opérateur retire ensuite l'introducteur en laissant le cathéter 6 en position.

L'invention permet donc d'obtenir un nécessaire de traitement 1 permettant une mise en place grandement simplifiée du cathéter 6 en position et limitant les risques d'infections.

La préparation du cathéter 6 est rapide à mettre en œuvre. En outre, elle demande moins de geste que les procédés connus dans l'état de la technique puisqu'il n'est pas nécessaire d'implanter plusieurs fois le cathéter 6.

En outre les risques d'infection sont diminués car le cathéter 6 n'est implanté qu'une fois dans le corps du patient. Il peut rester dans des conditions stériles jusqu'à son implantation.

De plus, le cathéter 6 mesurant précisément la longueur déterminée L_{D}, lors de sa mise en place, l'opérateur n'a pas besoin d'effectuer des compensations en augmentant la longueur de la partie émergée du cathéter. Une telle compensation induit un maintien plus délicat du cathéter et des risques d'infection. En outre, la mesure et la découpe étant précise, le cathéter 6 peut être mis en position à moins d'un centimètre de la zone ciblée.

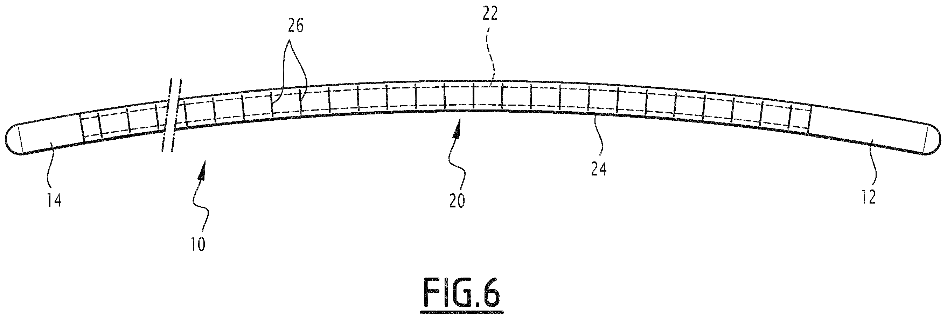

Dans une variante illustrée par la figure 6, le guide 10 ne présente pas de courbure 36. En outre, l'âme 22 ne comporte pas de ressort 23. L'âme 22 est un jonc métallique. L'âme 22 présente une forme cylindrique allongée. Les portions de l'âme 22 au niveau des extrémités distale 12 et proximale 14, sont non couvertes par la gaine isolante 24. Elles mesurent sensiblement 1 à 2 centimètres.

Un tel guide est avantageusement destiné au placement d'un cathéter central inséré périphériquement (PICC)

Le nécessaire de traitement 1 est un matériel dédié à la préparation de la mise en place du cathéter. Les dimensions et la nature du guide 10 peuvent donc être particulièrement adapté à cette application et supporté par un marquage CE.

En outre, la possibilité de couper le cathéter 6 au niveau de son extrémité distale 42 et non proximale 40, permet d'utiliser cette technique avec des chambres pré-connectées, des PICC, des CVC, ce qui favorise l'asepsie et facilite la tâche des praticiens et améliore la précision de la position de l'extrémité distale 42 du cathéter 6.

## Revendications

1. Nécessaire de traitement (1) comprenant :
- un guide (10) comportant une extrémité distale (12) et une extrémité proximale (14),
- un dispositif de détermination (16) de la position de l'extrémité distale (12) du guide (10) dans le corps d'un patient, et
- un cathéter (6) destiné à être inséré dans le corps du patient,
**caractérisé en ce que** le guide (10) est gradué entre son extrémité distale (12) et son extrémité proximale (14), les graduations (26) du guide (10) permettant de mesurer une longueur d'insertion du guide (10) dans le corps du patient et **en ce que** le cathéter (6) est propre à être découpé à une longueur déterminée en fonction de la longueur d'insertion, et **en ce que** le guide (10) comporte une portion centrale (20) entre l'extrémité distale (12) et l'extrémité proximale (14) couverte d'un matériau isolant électriquement, l'extrémité distale (12) et l'extrémité proximale (14) étant en matériau conducteur électriquement.

2. Nécessaire de traitement (1) selon la revendication 1, dans lequel le cathéter (6) est propre à être inséré dans le corps du patient, à la place du guide (10), après sa découpe.

3. Nécessaire de traitement (1) selon l'une quelconque des revendications 1 ou 2, dans lequel le dispositif de détermination (16) de la position de l'extrémité distale (12) du guide (10) dans le corps du patient comporte une sonde d'électrocardiogramme (34) reliée à l'extrémité proximale (14) du guide (10), le signal mesuré par la sonde (34) dépendant de la position de l'extrémité distale (12) du guide (10).

4. Nécessaire de traitement (1) selon l'une quelconque des revendications 1 à 3, dans lequel le diamètre externe du guide (10) est inférieur à 1,5 mm, notamment inférieur à 0,9 mm, avantageusement inférieur à 0,5 mm et, de préférence, inférieur à 0,4 mm.

5. Nécessaire de traitement (1) selon l'une quelconque des revendications 1 à 4, dans lequel le cathéter (6) comporte une extrémité distale (42) et une extrémité proximale (40), et le cathéter (6) est propre à être découpé par son extrémité distale (42).

6. Nécessaire de traitement (1) selon l'une quelconque des revendications 1 à 5, dans lequel le cathéter (6) comporte une extrémité distale (42) et une extrémité proximale (40) et le cathéter (6) comporte une chambre implantable (50) ou l'embase d'un cathéter central inséré périphériquement ou d'un cathéter veineux central raccordée à son extrémité proximale (40).

7. Nécessaire de traitement (1) selon l'une quelconque des revendications 1 à 6, dans lequel le guide (10) comporte une âme (22) au moins partiellement conductrice électriquement et une gaine externe isolante (24), les graduations (26) du guide (10) étant disposées sur la gaine externe (24) ou sur l'âme (22).

8. Guide (10) destiné à être inséré partiellement dans le corps d'un patient et à être raccordé à un dispositif de détermination (16) de la position de l'extrémité distale (12) du guide (10) dans le corps d'un patient, le guide (10) comportant une extrémité distale (12) et une extrémité proximale (14), **caractérisé en ce que** le guide (10) est gradué entre son extrémité distale (12) et son extrémité proximale (14), les graduations (26) du guide (10) permettant de mesurer à l'extérieur du corps du patient une longueur d'insertion du guide (10) dans le corps du patient, le guide comportant une portion centrale (20) entre l'extrémité distale (12) et l'extrémité proximale (14) couverte d'un matériau isolant électriquement, l'extrémité distale (12) et l'extrémité proximale (14) étant en matériau conducteur électriquement.

9. Dispositif de mesure (4) comprenant :
- un guide (10) destiné à être inséré partiellement dans le corps d'un patient, le guide comportant une extrémité distale (12) et une extrémité proximale (14),
- un dispositif de détermination (16) de la position de l'extrémité distale (12) du guide (10) dans le corps du patient propre à être raccordé au guide (10) ;
**caractérisé en ce que** le guide (10) est gradué entre son extrémité distale (12) et son extrémité proximale (14), les graduations (26) du guide (10) permettant de mesurer une longueur d'insertion, le guide comportant une portion centrale (20) entre l'extrémité distale (12) et l'extrémité proximale (14) couverte d'un matériau isolant électriquement, l'extrémité distale (12) et l'extrémité proximale (14) étant en matériau conducteur électriquement.

## Patentansprüche

1. Behandlungssatz (1), umfassend:
- eine Führung (10), die ein distales Ende (12) und ein proximales Ende (14) aufweist,
- eine Vorrichtung (16) zum Bestimmen der Position des distalen Endes (12) der Führung (10) in dem Körper eines Patienten, und
- einen Katheter (6), der vorgesehen ist, in den Körper des Patienten eingeführt zu werden,
**dadurch gekennzeichnet, dass** die Führung (10) zwischen ihrem distalen Ende (12) und ihrem proximalen Ende (14) mit einer Skala versehen ist, wobei die Skala (26) der Führung (10) ermöglicht, die Einführungslänge der Führung (10) in den Körper des Patienten zu messen, und dass der Katheter (6) geeignet ist, bei einer bestimmten Länge abhängig von der Einführungslänge abgeschnitten zu werden, und dass die Führung (10) einen Mittelabschnitt (20) zwischen dem distalen Ende (12) und dem proximalen Ende (14) aufweist, der mit einem elektrisch isolierenden Material bedeckt ist, wobei das distale Ende (12) und das proximale Ende (14) aus einem elektrisch leitenden Material bestehen.

2. Behandlungssatz (1) nach Anspruch 1, bei dem der Katheter (6) geeignet ist, in den Körper des Patienten nach seinem Zuschneiden anstelle der Führung (10) eingeführt zu werden.

3. Behandlungssatz (1) nach einem beliebigen der Ansprüche 1 oder 2, bei der die Vorrichtung (16) zum Bestimmen der Position des distalen Endes (12) der Führung (10) in dem Körper des Patienten eine Elektrokardiogrammsonde (34) aufweist, die mit dem proximalen Ende (14) der Führung (10) verbunden ist, wobei das von der Sonde (34) gemessene Signal von der Position des distalen Endes (12) der Führung (10) abhängt.

4. Behandlungssatz (1) nach einem beliebigen der Ansprüche 1 bis 3, bei dem der Außendurchmesser der Führung (10) kleiner als 1,5 mm, insbesondere kleiner als 0,9 mm, vorteilhafterweise kleiner als 0,5 mm, vorzugsweise kleiner als 0,4 mm ist.

5. Behandlungssatz (1) nach einem beliebigen der Ansprüche 1 bis 4, bei dem der Katheter (6) ein distales Ende (42) und ein proximales Ende (40) aufweist und der Katheter (6) geeignet ist, an seinem distalen Ende (42) abgeschnitten zu werden.

6. Behandlungssatz (1) nach einem beliebigen der Ansprüche 1 bis 5, bei dem der Katheter (6) ein distales Ende (42) und ein proximales Ende (40) aufweist und der Katheter eine implantierbare Kammer (50) oder den Sockel eines zentralen Katheters, der peripher eingeführt wird, oder einen zentralen Venenkatheter, der an sein proximales Ende (40) angeschlossen wird, aufweist.

7. Behandlungssatz (1) nach einem beliebigen der Ansprüche 1 bis 6, bei der die Führung (10) eine Seele (22), die mindestens teilweise elektrisch leitend ist, und eine isolierende Außenhülle (24) aufweist, wobei die Skala (26) der Führung (10) auf der Außenhülle (24) oder auf der Seele (22) angeordnet ist.

8. Führung (10), die vorgesehen ist, teilweise in den Körper eines Patienten eingeführt zu werden und mit einer Vorrichtung (16) zur Bestimmung der Position des distalen Endes (12) der Führung (10) in dem Körper eines Patienten verbunden zu werden, wobei die Führung (10) ein distales Ende (12) und ein proximales Ende (14) aufweist, **dadurch gekennzeichnet, dass** die Führung (10) zwischen ihrem distalen Ende (12) und ihrem proximalen Ende (14) mit einer Skala versehen ist, wobei die Skala (26) der Führung (10) ermöglicht, außerhalb des Körpers des Patienten eine Einführungslänge der Führung (10) in den Körper des Patienten zu messen, wobei die Führung einen Mittelabschnitt (20) zwischen dem distalen Ende (12) und dem proximalen Ende (14) aufweist, der mit einem elektrisch isolierenden Material bedeckt ist, wobei das distale Ende (12) und das proximale Ende (14) aus einem elektrisch leitenden Material bestehen.

9. Messvorrichtung (4), umfassend:
- einen Führung (10), die vorgesehen ist, teilweise in den Körper eines Patienten eingeführt zu werden, wobei die Führung ein distales Ende (12) und ein proximales Ende (14) aufweist,
- eine Vorrichtung (16) zum Bestimmen der Position des distalen Endes (12) der Führung (10) in dem Körper des Patienten, die geeignet ist, an die Führung (10) angeschlossen zu werden;
**dadurch gekennzeichnet, dass** die Führung (10) zwischen ihrem distalen Ende (12) und ihrem proximalen Ende (14) mit einer Skala versehen ist, wobei die Skala (26) der Führung (10) ermöglicht, eine Einführungslänge zu messen, wobei die Führung einen Mittelabschnitt (20) zwischen dem distalen Ende (12) und dem proximalen Ende (14) aufweist, der mit einem elektrisch isolierenden Material bedeckt ist, wobei das distale Ende (12) und das proximale Ende (14) aus einem elektrisch leitenden Material bestehen.

## Claims

1. A treatment kit (1) comprising:
- a guide (10) having a distal end (12) and a proximal end (14) ;
- a position determination device (16) for determining the position of the distal end (12) of the guide (10) in the body of a patient; and
- a catheter (6) intended to be inserted into the body of the patient;
**characterised in that** the guide (10) is graduated between its distal end (12) and its proximal end (14) the graduations (26) of the guide (10) making it possible to measure a length of insertion of the guide (10) into the body of the patient, and **in that** the catheter (6) is adapted so as to be cut to a predetermined length that is determined based on the length of insertion, and **in that** the guide (10) includes a central portion (20) between the distal end (12) and the proximal end (14) covered with an electrically insulating material, the distal end (12) and the proximal end (14) being made of an electrically conductive material.

2. A treatment kit (1) according to claim 1, in which the catheter (6) is adapted so as to be inserted into the body of the patient, instead of the guide (10) after it has been cut.

3. A treatment kit (1) according to claim 1 or 2, in which the position determination device (16) for determining the position of the distal end (12) of the guide (10) in the body of the patient comprises an electrocardiogram sensor probe (34) connected to the proximal end (14) of the guide (10) with the signal measured by the probe (34) being dependent on the position of the distal end (12) of the guide (10).

4. A treatment kit (1) according to any of claims 1 to 3, in which the external diameter of the guide (10) is less than 1.5 mm, in particular less than 0.9 mm, advantageously less than 0.5 mm and, preferably, less than 0.4 mm.

5. A treatment kit (1) according to any of claims 1 to 4, in which the catheter (6) has a distal end (42) and a proximal end (14) and the catheter (6) is adapted so as to be cut at its distal end (42).

6. A treatment kit (1) according to any of claims 1 to 5, in which the catheter (6) has a distal end (42) and a proximal end (40) and the catheter (6) includes an implantable port (50) or the base of a peripherally inserted central catheter (6) or of a central venous catheter connected to its proximal end (40).

7. A treatment kit (1) according to any of claims 1 to 6, in which the guide (10) includes a core (22) that is at least partially electrically conductive and an insulating external sheath (24), with the graduations (26) of the guide (10) being arranged over the external sheath (24) or over the core (22).

8. A guide (10) designed to be inserted partially into the body of a patient and to be connected to a position determination device (16) for determining the position of the distal end (12) of the guide (10) in the body of a patient, with the guide (10) having a distal end (12) and a proximal end (14), **characterised in that** the guide (10) is graduated between its distal end (12) and its proximal end (14), the graduations (26) of the guide (10) making it possible to measure from the exterior of the body of the patient a length of insertion of the guide (10) into the body of the patient, the guide (10) including a central portion (20) between the distal end (12) and the proximal end (14) that is covered with an electrically insulating material, with the distal end (12) and the proximal end (14) being made of an electrically conductive material.

9. A measuring device comprising:
- a guide (10) designed to be inserted partially into the body of a patient, with the guide (10) having a distal end (12) and a proximal end (14),
- a position determination device (16) for determining the position of the distal end (12) of the guide (10) in the body of the patient, that is capable of being connected to the guide (10);
**characterised in that** the guide (10) is graduated between its distal end (12) and the proximal end (14), the graduations (26) of the guide (10) making it possible to measure a length of insertion, the guide (10) including a central portion (20) between the distal end (12) and the proximal end (14) that is covered with an electrically insulating material, with the distal end (12) and the proximal end (14) being made of an electrically conductive material..
